(19) **Europäisches Patentamt / European Patent Office / Office européen des brevets**

(11)  **EP 2 950 667 B1**

(12)  **FASCICULE DE BREVET EUROPEEN**

(45) Date de publication et mention de la délivrance du brevet:
**22.11.2017 Bulletin 2017/47**

(21) Numéro de dépôt: **14706981.9**

(22) Date de dépôt: **22.01.2014**

(51) Int Cl.:
***A23L 33/10*** *(2016.01)*

(86) Numéro de dépôt international:
**PCT/TN2014/000001**

(87) Numéro de publication internationale:
**WO 2014/120099 (07.08.2014 Gazette 2014/32)**

(54) **COMPLÉMENT ALIMENTAIRE OMÉGA 3 À BASE D'HUILE MARINE, AMÉLIORÉ**

VERBESSERTE OMEGA-3 NAHRUNGSERGÄNZUNGSMITTEL AUF BASIS VON FISCHÖL

IMPROVED OMEGA-3 FOOD SUPPLEMENT BASED ON MARINE OIL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.01.2013 TN 20130036**

(43) Date de publication de la demande:
**09.12.2015 Bulletin 2015/50**

(73) Titulaire: **GUETARI, Sami**
**Tunis (TN)**

(72) Inventeur: **GUETARI, Sami**
**Tunis (TN)**

(74) Mandataire: **Demulsant, Xavier**
**Dejade & Biset**
**35, rue de Châteaudun**
**75009 Paris (FR)**

(56) Documents cités:
| | |
|---|---|
| EP-A1- 2 279 667 | EP-A2- 0 304 115 |
| WO-A1-2004/047554 | WO-A1-2006/118463 |
| WO-A1-2009/120091 | FR-A1- 2 911 252 |
| US-A1- 2012 171 285 | |

**Description**

La présente invention se rapporte à l'utilisation non thérapeutique d'au moins un complément alimentaire à base d'huile marine (poisson, coproduits de poisson, mollusques, krill, algues, micro algues) enrichi en huile végétale et/ou marine, en tant que principe actif dans une composition alimentaire ou parapharmaceutique.

Les omégas 3 sont des acides gras poly insaturés essentiels, car l'organisme en a besoin pour son bon fonctionnement, mais il ne peut les synthétiser qu'en très faible quantité. L'alimentation est donc la source primordiale d'apport. Ils se présentent dans un état liquide et huileux. A nombre égal de carbone, ils présentent toujours des points de fusions plus bas que les acides gras saturés.

Les acides gras mono insaturés des familles (n-7) et (n-9) tels que l'acide palmitoléique C16 : 1 (n-7) et l'acide oléique C18 : 1 (n-9) très présents dans l'huile d'olive, jouent le rôle de précurseur des autres acides gras poly insaturés.

L'acide-alpha-linolénique : C18 :3 n-3 et l'acide linoléique : C18 :2 n-6, sont dits acides gras essentiels, ils sont métabolisés de façon à synthétiser des acides gras poly insaturés (AGPI). WO 2006/118463 A1 décrit un complément alimentaire contenant des omégas 3 caractérisé en ce qu'il contient une combinaison d'huile marine (huile de phoque) et d'huile d'olive pressée à froid. WO 2009/120091 A1 décrit une formule de boisson comprenant une huile oméga 3 fraîche d'origine marine et des antioxydants. Le marché des oméga-3 ne cesse de se développer, tant au niveau des aliments et boissons que des compléments alimentaires. En 2010, le marché des aliments et boissons riches en oméga-3 représentait 4 milliards de dollars, contre 1,3 milliards de dollars pour les compléments alimentaires. Selon le centre d'analyse de marché Packaged Facts, on s'attend à une croissance de 15 à 20% d'ici 2015.
Source : http://www.nutraingredients.com/Consumer-Trends/Global-omega-3-market-setfor-ongoing-15-20-growth-Report

Les omégas 3 sont utilisés dans l'industrie agroalimentaire qui lance régulièrement sur le marché de nouveaux produits enrichis en EPA et en DHA (Yaourts, laits, margarines, oeufs...) pour répondre aux demandes du consommateur à la recherche de santé et du bien-être.

Ces produits sont fabriqués à base d'huile marine (poisson, coproduits de poisson, krill, algues, micro algues). La question qui se pose est de vérifier si la composition de ces huiles marines est suffisamment équilibrée en acides gras.

Pour répondre à cette problématique, nous avons utilisé un indice : le score lipidique de prévention (SLP), qui est un indice calculé pour chaque aliment, il tient compte de sa teneur en lipides totaux (L) et de sa composition en AGS, AGMI et AGPI. Le SLP permet de classer et de choisir les aliments pour une meilleure prévention des maladies cardio-vasculaires. Le SLP est corrélé de façon hautement significative avec les paramètres qui figurent dans des aliments gras.

Le SLP est calculé en appliquant la formule suivante :

$$SLP = L + 2\ AGS - 1{,}184\ AGMI - 0{,}4\ AGPI$$

Cette équation révèle que si les proportions en AGS, AGMI et AGPI d'un aliment sont égales respectivement à 33%, 50% et 17% alors son SLP est égal à L et l'aliment est jugé correctement équilibré en acides gras. Si la proportion des AGS dépasse 33%, le SLP est supérieur à L, inversement si les AGMI ou AGPI dépassent respectivement 50% ou 17%, le SLP est inférieur à L. on doit donc compenser les aliments à fort SLP, par des aliments à faible SLP.

A titre d'exemples, nous allons étudier la composition lipidique des huiles extraites à partir des coproduits du thon rouge engraissé, à savoir l'huile du mésentère, du foie, de la vessie natatoire, de l'estomac, du coeur et des reins.

**Exemple 1 : Les acides gras du mésentère**

La quantité d'acides gras totaux présente dans le mésentère du thon rouge engraissé est égale à 43,25 g/100g de MF (tableau 1).

Les acides gras saturés représentent 32,02% de cette quantité. Les mono insaturés 29,57% Les poly insaturés (hors EPA et DHA) 9,5%. L'EPA 14,17% et le DHA 7,91% (figure 1).

Le rapport AGI/AGS est égal à 1,91. Le rapport AGPI/AGS est égal à 0,98.
Le rapport n-3/n-6 est égal à 11,85 et le rapport n-6/n-3 est égal à 0,084.

Tableau 1 : Composition en acides gras du mésentère du thon rouge engraissé.

| N° | TR | AG | % |
|----|------|-------|-------|
| 1 | 4,02 | C14:0 | 7,347 |
| 2 | 4,36 | NI | 0,29 |

(suite)

| N° | TR | AG | % |
|---|---|---|---|
| 3 | 4,68 | NI | 0,74 |
| 4 | 5,47 | C16:0 | 20,32 |
| 5 | 5,66 | C16:1n-7 | 8,46 |
| 6 | 5,95 | NI | 0,51 |
| 7 | 6,17 | NI | 1,07 |
| 8 | 6,26 | NI | 0,28 |
| 9 | 6,61 | *C16:4 n-1* | 0,33 |
| 10 | 7,08 | C18:0 | 1,28 |
| 11 | 7,61 | C18:0 | 4,37 |
| 12 | 7,82 | C18:1 n-9 | 13,7 |
| 13 | 7,89 | C18:1 n-7 | 3,27 |
| 14 | 8,22 | C18:2 n-4 | 0,39 |
| 15 | 8,40 | C18:2 n-6 | 1,06 |
| 16 | 8,70 | C18:3 n-6 | 0,53 |
| 17 | 9,12 | NI | 0,29 |
| 18 | 9,34 | C18: 3 n-3 | 0,78 |
| 19 | 9,81 | C18: 4 n-3 | 2,15 |
| 20 | 10,01 | C18: 5 n-3 | 0,37 |
| 21 | 10,81 | NI | 0,56 |
| 22 | 11,09 | C20:1 n-9 | 2,48 |
| 23 | 11,58 | NI | 0,42 |
| 24 | 12,84 | C20:4 n-3 | 0,95 |
| 25 | 13,77 | C20:4 n-6 | 0,81 |
| 26 | 14,24 | C20:5 n-3 | 14,17 |
| 27 | 15,37 | C22:1 n-11 | 1,64 |
| 29 | 19,43 | C22:5 n-3 | 2,13 |
| 30 | 20,14 | C22:6 n-3 | 7,91 |
| ❶NI | - | - | 4.05 |

(N° : numéro du pic ; TR : temps de rétention ; AG : acide gras ; % : pourcentage moyen de l'acide gras considéré par rapport aux AGT ; NI : acide gras non identifié ; ❶NI : Somme des % moyens d'acides gras non identifiés ; AGT : % d'acides gras totaux présents dans 100 g de matière fraîche).

Fig. 1 : % des différents types d'acides gras contenus dans le Mésentère par rapport à la quantité d'acides gras totaux.

Exemple 2 : Les acides gras du foie

La quantité moyenne d'acides gras totaux présente dans le foie du thon rouge engraissé est égale à 13 g/100g de MF (tableau 2). Les AGS représentent 32,18% de cette quantité. Les AGMI 34,38% et les AGPI (hors EPA et DHA) 9,53%. L'EPA 12,82% et le DHA 6,6% (figure 2).

Le rapport AGI/AGS est égal à 1,96. Le rapport AGPI/AGS est égal à 0,90. Le rapport n-3/n-6 est égal à 10,31 et le rapport n-6/n-3 est égal à 0,096.

Tableau 2: Composition en acides gras du foie du thon rouge engraissé.

| N° | TR | AG | % |
|----|----|----|----|
| 1 | 4,02 | C14:0 | 3.64 |
| 2 | 4,36 | NI | 0.13 |
| 3 | 4,68 | NI | 0.43 |
| 4 | 5,08 | NI | 0.18 |
| 5 | 5,49 | C16 :0 | 20.87 |
| 6 | 5,67 | C18:1 n-7 | 6.52 |
| 7 | 5,95 | NI | 0.55 |
| 8 | 6,17 | NI | 0.59 |
| 9 | 6,64 | C16:4 n-1 | 0.52 |
| 10 | 7,09 | C18:0 | 7.67 |
| 11 | 7,65 | C18:0 | 7.67 |
| 12 | 7,87 | C18:1 n-9 | 19.24 |
| 13 | 7,94 | C18:1 n-7 | 4.16 |
| 14 | 8,24 | C18:2 n-4 | 0.49 |
| 15 | 8,42 | C18:2 n-6 | 0.81 |
| 16 | 8,71 | C18:3 n-6 | 0.73 |
| 17 | 9,13 | NI | 0.38 |
| 18 | 9,35 | C18:3 n-3 | 0.44 |
| 19 | 9,82 | C18:4 n-3 | 1.07 |
| 20 | 10,01 | C18:5 n-3 | 0.31 |
| 21 | 10,83 | NI | 0.26 |
| 22 | 11,09 | C20:1 n-9 | 2.76 |
| 23 | 11,59 | NI | 0.72 |
| 24 | 12,84 | C20:4 n-3 | 0.86 |
| 25 | 13,78 | C20:4 n-6 | 0.93 |
| 26 | 14,28 | C20:5 n-3 | 12.82 |
| 27 | 15,39 | C22:1 n-11 | 1.70 |
| 28 | 16,85 | NI | 0.828 |
| 29 | 19,43 | C22:5 n-3 | 3.37 |
| 30 | 20,17 | C22:6 n-3 | 6.60 |
| ❶ NI | - | - | 4,06 |

(N° : numéro du pic ; TR : temps de rétention; AG : acide gras ; % : pourcentage moyen de l'acide gras considéré par rapport aux AGT ; NI : acide gras non identifié ; ❶ NI : Somme des % moyens d'acides gras non identifiés ; AGT : % d'acides gras totaux présents dans 100 g de matière fraîche).

Fig. 2 : % des différents types d'acides gras contenus dans le Foie par rapport à la quantité d'acides gras totaux.

Exemple 3 : Les acides gras de la vessie natatoire

La quantité d'acides gras totaux présente dans la vessie natatoire du thon rouge engraissé est égale à 28,5 g/100g de MF (tableau 3).

Les acides gras saturés représentent 32,45%. Les mono insaturés 28,45%. Les poly insaturés (hors EPA et DHA) 9,6%. L'EPA 13,57% et le DHA 8,86 (figure 3).

Le rapport AGI/AGS est égal à 1,86. Le rapport AGPI/AGS est égal à 0,98. Le rapport n-3/n-6 est égal à 10,56 et le rapport n-6/n-3 est égal à 0,094.

Tableau 3 : Composition en acides gras de la vessie natatoire du thon rouge engraissé.

| N° | TR | AG | % |
|---|---|---|---|
| 1 | 4,03 | C14:0 | 8,50 |
| 2 | 4,36 | NI | 0,74 |
| 3 | 4,69 | NI | 0,71 |
| 4 | 5,48 | NI | 19,35 |
| 5 | 5,67 | C16:0 | 8,17 |
| 6 | 5,95 | C16:1 n-7 | 0,47 |
| 7 | 6,17 | NI | 1,05 |
| 8 | 6,26 | NI | 0,25 |
| 9 | 6,62 | C16:4 n-1 | 0,27 |
| 10 | 7,08 | C18 :0 | 1,24 |
| 11 | 7,62 | C18:0 | 4,60 |
| 12 | 7,82 | C18:1 n-9 | 12,39 |
| 13 | 7,88 | C18:1 n-7 | 4,04 |
| 14 | 8,22 | C18:2 n-4 | 0,19 |
| 15 | 8,40 | C18:2 n-6 | 0,95 |
| 16 | 8,71 | C18:3 n-6 | 0,51 |
| 17 | 9,12 | NI | 0,54 |
| 18 | 9,34 | C18:3 n-3 | 0,97 |
| 19 | 9,81 | C18:4 n-3 | 2,05 |
| 20 | 10,01 | C18:5 n-3 | 0,32 |
| 21 | 10,82 | NI | 0,28 |
| 22 | 11,08 | C20:1 n-9 | 2,42 |
| 23 | 11,57 | NI | 0,39 |
| 24 | 12,83 | C20:4 n-3 | 1,02 |
| 25 | 13,78 | C20:4 n-6 | 1,27 |
| 26 | 14,24 | C20:5 n-3 | 13,57 |

(suite)

| N° | TR | AG | % |
|----|------|-----------|------|
| 27 | 15,39 | C22:1 n-11 | 1,43 |
| 28 | 16,86 | NI | 1,34 |
| 29 | 19,43 | C22:5 n-3 | 2,05 |
| 30 | 20,15 | C22:6 n-3 | 8,86 |
| ❶NI | - | - | 5,77 |

(N° : numéro du pic ; TR : temps de rétention; AG : acide gras ; % : pourcentage moyen de l'acide gras considéré par rapport aux AGT ; NI : acide gras non identifié ; ❶NI : Somme des % moyens d'acides gras non identifiés ; AGT : % d'acides gras totaux présents dans 100 g de matière fraîche).

Fig. 3 : % des différents types d'acides gras contenus dans la vessie natatoire par rapport à la quantité d'acides gras totaux.

**Exemple 4 : Les acides gras du coeur**

La quantité d'acides gras totaux présente dans le coeur du thon rouge engraissé est égale à 1,65g/100g de MF (tableau 4).

Les acides gras saturés représentent 36,27% de cette quantité. Les mono insaturés 26,9%. Les poly insaturés (hors EPA et DHA) 8,06%. L'EPA 10,82 et le DHA 14,97 (figure 4).

Le rapport AGI/AGS est égal à 1,67. Le rapport AGPI/AGS est égal à 0,93. Le rapport n-3/n-6 est égal à 17,88 et le rapport n-6/n-3 est égal à 0,055.

Tableau 4 : Composition en acides gras du coeur du thon rouge engraissé.

| N° | TR | AG | % |
|----|------|-----------|-------|
| 1 | 4,03 | C14 :0 | 3,83 |
| 2 | 4,37 | NI | 0,16 |
| 3 | 4,69 | NI | 0,47 |
| 4 | 5,09 | NI | 0,16 |
| 5 | 5,50 | C16 :0 | 22,56 |
| 6 | 5,68 | C16:1 n-7 | 5,22 |
| 7 | 5,96 | NI | 0,45 |
| 8 | 6,18 | NI | 0,55 |
| 9 | 6,71 | C16:4 n-1 | 0,38 |
| 10 | 7,10 | C18 :0 | 3,64 |
| 11 | 7,66 | C18:0 | 9,88 |
| 12 | 7,86 | C18:1 n-9 | 12,12 |
| 13 | 7,91 | C18:1 n-7 | 4,62 |
| 14 | 8,25 | C18:2 n-4 | 0,23 |

(suite)

| N° | TR | AG | % |
|---|---|---|---|
| 15 | 8,43 | C18:2 n-6 | 0,77 |
| 16 | 8,72 | C18:3 n-6 | 0,43 |
| 17 | 9,10 | NI | 0,47 |
| 18 | 9,35 | C18:3 n-3 | 0,47 |
| 19 | 9,82 | C18:4 n-3 | 0,83 |
| 20 | 10,02 | C18:5 n-3 | 0,15 |
| 22 | 11,09 | C20:1 n-9 | 2,25 |
| 23 | 11,57 | NI | 0,89 |
| 24 | 12,83 | C20:4 n-3 | 2,07 |
| 25 | 13,77 | C20:4 n-8 | 0,56 |
| 26 | 14,27 | C20:5 n-3 | 10,82 |
| 27 | 15,37 | C22:1 n-11 | 1,67 |
| 29 | 19,43 | C22:5 n-3 | 2,17 |
| 30 | 20,19 | C22:6 n-3 | 14,97 |
| ❶NI | - | - | 3,65 |

(N° : numéro du pic ; TR : temps de rétention ; AG : acide gras ; % : pourcentage moyen de l'acide gras considéré par rapport aux AGT; NI : acide gras non identifié; ❶NI : Somme des % moyens des acides gras non identifiés ; AGT : % des acides gras totaux présents dans 100 g de matière fraîche).

Fig. 4 : % des différents types d'acides gras contenus dans le cœur par rapport à la quantité d'acides gras totaux.

**Exemple 5 : Les acides gras de l'estomac**

La quantité d'acides gras totaux présente dans l'estomac du thon rouge engraissé est égale à 2,65g/100g de MF (tableau 5).

Les acides gras saturés représentent 36,13% de cette quantité. Les mono insaturés 22,03%. Les poly insaturés (hors EPA et DHA) 11,15%. l'EPA 13,68% et le DHA 12,5% (figure 5).

Le rapport AGI/AGS est égal à 1,64. Le rapport AGPI/AGS est égal à 1,13. Le rapport n-3/n-6 est égal à 18,83 et le rapport n-6/n-3 est égal à 0,053.

Tableau 5 : Composition en acides gras de l'estomac du thon rouge engraissé.

| N° | TR | AG | % |
|---|---|---|---|
| 1 | 4,02 | C14 :0 | 4,2 |
| 2 | 4,28 | NI | 0,82 |

(suite)

| N° | TR | AG | % |
|---|---|---|---|
| 3 | 4,69 | NI | 0,80 |
| 4 | 5,08 | NI | 0,34 |
| 5 | 5,47 | C16 :0 | 21,64 |
| 6 | 5,66 | C16:1 n-7 | 4,54 |
| 7 | 5,97 | NI | 0,20 |
| 8 | 6,17 | NI | 0,35 |
| 9 | 6,74 | C16:4 n-1 | 0,29 |
| 10 | 7,09 | C18 :0 | 0,34 |
| 11 | 7,62 | C18 :0 | 10,26 |
| 12 | 7,82 | C18:1 n-9 | 11,25 |
| 13 | 7,88 | C18:1 n-7 | 4,02 |
| 14 | 8,22 | C18:2 n-4 | 0,14 |
| 15 | 8,40 | C18:2 n-6 | 0,91 |
| 16 | 8,70 | C18:3 n-6 | 0,41 |
| 17 | 9,10 | NI | 0,40 |
| 18 | 9,35 | C18:3 n-3 | 0,49 |
| 19 | 9,81 | C18:4 n-3 | 0,75 |
| 20 | 10,01 | C18:5 n-3 | 0,29 |
| 21 | 10,81 | NI | 0,22 |
| 22 | 11,09 | C20:1 n-9 | 1,56 |
| 23 | 11,57 | NI | 0,24 |
| 24 | 12,83 | C20:4 n-3 | 4,77 |
| 25 | 13,77 | C20:4 n-6 | 0,54 |
| 26 | 14,24 | C20:5 n-3 | 13,68 |
| 27 | 15,38 | C22:1 n-11 | 0,66 |
| 28 | 16,84 | NI | 0,64 |
| 29 | 19,41 | C22:5 n-3 | 2,56 |
| 30 | 20,13 | C22:6 n-3 | 12,50 |
| ❶NI | - | - | 4,35 |

(N° : numéro du pic ; TR : temps de rétention; AG : acide gras ; % : pourcentage moyen de l'acide gras considéré par rapport aux AGT ; NI : acide gras non identifié ; ❶NI : Somme des % moyens des acides gras non identifiés ; AGT : % des acides gras totaux présents dans 100 g de matière fraîche).

Fig. 5 : % des différents types d'acides gras de l'estomac par rapport à la quantité d'acides gras totaux.

**Exemple 6 : Les acides gras des reins**

La quantité d'acides gras totaux présente dans le rein du thon rouge engraissé est égale à 0,94 g/100g de MF (tableau 6).

Les acides gras saturés représentent 29,32% de cette quantité. Les mono insaturés 32,39%. Les poly insaturés (hors EPA et DHA) 11,71%. L'EPA 11,37% et le DHA 8,86% (figure 6).

Le rapport AGI/AGS est égal à 2,17. Le rapport AGPI/AGS est égal à 1,07. Le rapport n-3/n-6 est égal à 13,1 et le rapport n-6/n-3 est égal à 0,076.

Tableau 6 : Composition en acides gras du rein du thon rouge engraissé.

| N° | TR | AG | % |
|---|---|---|---|
| 1 | 4,03 | C14 :0 | 4,54 |
| 2 | 4,36 | NI | 0,41 |
| 3 | 4,69 | NI | 0,51 |
| 4 | 5,08 | NI | 0,05 |
| 5 | 5,48 | C16 :0 | 18,73 |
| 6 | 5,67 | C16:1 n-7 | 6,60 |
| 7 | 5,95 | NI | 0,44 |
| 8 | 6,17 | NI | 0,71 |
| 9 | 6,63 | C16:4 n-1 | 0,43 |
| 10 | 7,09 | C18 :0 | 0,59 |
| 11 | 7,63 | C18 :0 | 6,05 |
| 12 | 7,84 | C18 :1 n-9 | 15,37 |
| 13 | 7,89 | C18 :1 n-7 | 4,66 |
| 14 | 8,23 | C18 :2 n-4 | 0,33 |
| 15 | 8,41 | C18 :2 n-6 | 0,84 |
| 16 | 8,72 | C18:3n-6 | 0,51 |
| 17 | 9,13 | NI | 0,32 |
| 18 | 9,30 | C18 :3 n-3 | 0,64 |
| 19 | 9,82 | C18 :4 n-3 | 1,32 |
| 20 | 10,02 | C18 :5 n-3 | 0,33 |
| 21 | 10,83 | NI | 0,59 |
| 22 | 11,10 | C20 :1 n-9 | 3,81 |
| 23 | 11,60 | NI | 0,89 |
| 24 | 12,85 | C20 :4 n-3 | 0,88 |
| 25 | 13,78 | C20 :4 n-6 | 0,86 |
| 26 | 14,26 | C20 :5 n-3 | 11,37 |
| 27 | 15,39 | C22 :1 n-11 | 1,95 |
| 28 | 16,85 | NI | 1,69 |
| 29 | 19,44 | C22 :5 n-3 | 5,57 |
| 30 | 20,16 | C22 :6 n-3 | 8,86 |
| ❶NI | - | - | 5,61 |

(N° : numéro du pic ; TR : temps de rétention ; AG : acide gras ; % : pourcentage moyen de l'acide gras considéré par rapport aux AGT ; NI : acide gras non identifié ; ❶NI : Somme des % moyens des acides gras non identifiés ; AGT : % des acides gras totaux présents dans 100 g de matière fraîche).

Fig. 6 : % des différents types d'acides gras contenus dans
le rein par rapport à la quantité des acides gras totaux.

## Récapitulation et conclusion

En utilisant les compositions en acides gras de ces six exemples, nous avons calculé le Score Lipidique de Prévention de chaque huile extraite à partir des coproduits du thon rouge engraissé. Les résultats sont mentionnés dans le tableau (7).

Tableau 7 : Les SLP relatifs aux huiles extraites à partir des coproduits issus de l'engraissement du thon rouge

| (100g d'huile) | L | SLP |
|---|---|---|
| Mésentère | 100 | 114,7 |
| Vessie natatoire | 100 | 116,1 |
| Foie | 100 | 110,4 |
| Estomac | 100 | 129,5 |
| Coeur | 100 | 125,7 |
| Rein | 100 | 105,2 |

D'après ces résultats, les SLP des huiles extraites à partir des coproduits issus de l'engraissement du thon rouge sont supérieurs à la quantité de lipides totaux (L), on doit donc compenser ces élévations par des aliments à SLP faible. Etant donné qu'elles sont des huiles, la teneur en lipides totaux dans chacune de ces 100 g d'huile est, comme indiqué dans le tableau ci-dessus, égale à 100.

Dans ce contexte, nous avons réfléchi à l'huile d'olive tunisienne, dont les proportions des différents types d'acides gras à travers sept variétés d'olive locale ont été étudiées par Dhifi en 2005 (tableau 8). Cet auteur avance les proportions suivantes :

Tableau 8 : Les proportions des différents types d'acides gras présentes dans l'huile extraite à partir de sept variétés d'olive tunisiennes.

| (100g d'huile) | Chetoui | Chemlali | Chemchali | Sayali | Meski | Zalmati | Oueslati |
|---|---|---|---|---|---|---|---|
| AGS (%) | 9,3 | 20,1 | 16,9 | 13,1 | 14,1 | 20,0 | 14,1 |
| AGMI (%) | 71,2 | 58,9 | 62,9 | 81,4 | 61,2 | 59,4 | 61,2 |
| AGPI (%) | 19,5 | 21,2 | 20,2 | 5,8 | 24,7 | 20,5 | 24,7 |

A partir de ces teneurs, nous avons calculé le score lipidique de prévention (SLP) de chacune de ces huiles. Les résultats sont consignés dans le tableau 9 :

Tableau 9 : Les scores lipidiques de prévention relatifs aux différentes variétés d'huiles tunisiennes.

| (100g d'huile) | Chetoui | Chemlali | Chemchali | Sayali | Meski | Zalmati | Oueslati |
|---|---|---|---|---|---|---|---|
| SLP | 26.5 | 62 | 51.2 | 27.5 | 45.8 | 61.4 | 45.8 |
| L | 100 | 100 | 100 | 100 | 100 | 100 | 100 |

Les valeurs du tableau (9) montrent que les SLP de toutes les huiles d'olive tunisiennes sont largement inférieurs à la quantité de lipides totaux que renferment ces huiles. Ils représentent en effet des aliments à score lipidique faible susceptibles d'être utilisées pour compenser les SLP élevés des huiles extraites à partir des coproduits du thon rouge engraissé.

D'après Dhifi (2005), la variété Chemlali constitue la quasi-totalité de l'oliveraie des régions du Centre, du Sahel et du Sud. Le rendement en huile est de 20 à 22%. Cette forte production est liée à la texture du sol, qui permet aux arbres de développer un très important système racinaire. Pour ces raisons, nous proposons de choisir l'huile de cette variété pour compenser les SLP élevés des huiles extraites à partir des coproduits du thon rouge engraissé.

En conclusion, afin de fabriquer un aliment correctement équilibré en acides gras, à Score Lipidique de Prévention optimisé, 1g de produit fini (complément alimentaire à base d'huile de coproduits de poisson) doit comporter une quantité bien déterminée d'huile d'olive de la variété Chemlali, ceci est détaillé dans le tableau 10 :

**Tableau 10 : les proportions d'huile d'olive de la variété Chemlali que doit Comporter 1g de produit fini.**

| huiles | Mésentère | Vessie natatoire | Foie | Estomac | Coeur | Rein |
|---|---|---|---|---|---|---|
| % d'huile (Chemlali) | 23,7 | 26 | 16,7 | 47,6 | 41,4 | 8,3 |

Ainsi, un complément alimentaire de 1g composé

- de 76.3% en poids de l'huile du mésentère et de 23.7 % en poids de l'huile d'olive de la variété Chemlali ; ou
- de 74 % en poids de l'huile de la vessie natatoire et de 26 % en poids de l'huile d'olive de la variété Chemlali; ou
- de 83.3% en poids de l'huile de foie et de 16.7 % en poids de l'huile d'olive de la variété Chemlali; ou
- de 52.4 % en poids de l'huile de l'estomac et de 47.6 % en poids de l'huile d'olive de la variété Chemlali ; ou
- de 58.6 % en poids de l'huile de coeur et de 41.4 % en poids de l'huile d'olive de la variété Chemlali ; ou
- de 91.7 % en poids de l'huile de rein et de 8.3 % en poids de l'huile d'olive de la variété Chemlali

est équilibré en acides gras. Autrement dit, le score lipidique de prévention SLP du mélange de la première huile et de la deuxième huile est égal à la teneur en lipides totaux L dans ce mélange.

Il est important de souligner que cette invention n'est pas spécifique au mélange d'huile d'olive et des huiles extraites à partir des coproduits issus de l'engraissement du thon rouge. Il est possible d'utiliser différents types d'huiles marines (poisson, coproduits de poisson, mollusques, coproduits de mollusques, krill, algues, micro algues) et les mélanger avec n'importe quel type d'huile végétale et/ou marine (à score lipidique de prévention faible ou fort), à des proportions bien déterminées, afin d'obtenir un produit à Score lipidique de prévention optimal, correctement équilibré en acides gras, qui peut être prescrit dans le cadre d'un régime alimentaire préventif et/ou curatif des hypercholestérolémies et des perturbations lipoprotéiniques qui conduisent à l'athérosclérose et par là aux maladies cardiovasculaires.

A titre d'exemples, le tableau n° 11 précise la composition en acides gras ainsi que les valeurs des Scores Lipidiques de Prévention de quelques huiles végétales et marines.

Tableau 11 : Composition en acides gras (en % du total des acides gras) et Scores Lipidiques de Prévention de quelques huiles végétales et marines

| | AGS | AGMI | AGPI | SLP |
|---|---|---|---|---|
| huile de Nigelle (cumin noir) | 11,93 | 23 | 56,2 | 74,1 |
| huile de mais | 13,5 | 28,9 | 57,6 | 69.7 |
| huile d'amande douce | 8 | 70 | 17 | 26,3 |
| huile de lin | 9 | 20 | 66 | 67,9 |
| Huile d'arachide | 21 | 57 | 22 | 65,7 |
| huile de noisette | 7 | 78 | 10 | 17,6 |

(suite)

| | AGS | AGMI | AGPI | SLP |
|---|---|---|---|---|
| huile de tournesol | 14 | 22 | 64 | 76,3 |
| huile d'olive | 12 | 81 | 7 | 25,9 |
| huile de canola | 6 | 56 | 33 | 32,4 |
| huile de noix | 9 | 23 | 63 | 65,5 |
| huile de krill | 28,5 | 19,5 | 27,5 | 122,9 |
| huile de chinchard | 30,5 | 25,5 | 40,5 | 114,6 |
| huile de sardines | 40,5 | 37,1 | 45,5 | 118,9 |
| huile d'anchois | 34 | 37,5 | 61,5 | 99,0 |
| huile de calamar | 24 | 49,5 | 62 | 64,6 |
| Huile de foie de morue | 22,6 | 46,71 | 22,54 | 80,9 |

Les résultats du tableau 11 montrent bien qu'il existe des huiles marines à Score Lipidiques de Prévention faible, telles que l'huile de calamar et l'huile de foie de morue. Ces dernières sont susceptibles d'être utilisées individuellement ou en mélange avec des huiles végétales afin d'optimiser les Scores Lipidiques de Prévention de la majorité des huiles marines à Score Lipidique de Prévention fort.

**Revendications**

1.  Un complément alimentaire comprenant

    - une première huile ayant un premier score lipidique de prévention supérieur à la teneur en lipides totaux dans cette première huile, cette première huile étant une huile marine ;
    - une deuxième huile ayant un deuxième score lipidique de prévention inférieur à la teneur en lipides totaux dans cette deuxième huile, cette deuxième huile étant une huile végétale et/ou marine ;

    ce complément alimentaire étant **caractérisé en ce que** le score lipidique de prévention du mélange de la première huile et de la deuxième huile est sensiblement égale à la teneur, dans ce mélange, en lipides totaux.

2.  Le complément alimentaire de la revendication précédente, dans lequel l'huile marine est choisie parmi la liste comprenant l'huile extraite à partir de coproduits du thon rouge engraissé, l'huile de krill, l'huile de chinchard, l'huile de sardine.

3.  Le complément alimentaire de la revendication précédente, dans lequel l'huile marine extraite à partir de coproduits du thon rouge engraissé est choisie parmi la liste comprenant l'huile du mésentère, l'huile du foie, l'huile de la vessie natatoire, l'huile de l'estomac, l'huile du coeur, l'huile des reins.

4.  Le complément alimentaire de l'une quelconque des revendications précédentes, dans lequel la deuxième huile comprend

    - une huile végétale choisie parmi la liste comprenant l'huile d'olive, l'huile de nigelle, l'huile de mais, l'huile d'amande douce, l'huile de lin, l'huile d'arachide, l'huile de noisette, l'huile de tournesol, l'huile de canola, l'huile de noix; et/ou
    - une huile marine choisie parmi la liste comprenant l'huile de calamar, l'huile de foie de morue, l'huile d'anchois.

5.  Le complément alimentaire de la revendication précédente, dans lequel l'huile d'olive est choisie parmi la liste comprenant l'huile d'olive de la variété Chetoui, l'huile d'olive de la variété Chemlali, l'huile d'olive de la variété Chemchali, l'huile d'olive de la variété Sayali, l'huile d'olive de la variété Meski, l'huile d'olive de la variété Zalmati, l'huile d'olive de la variété Oueslati.

6. Le complément alimentaire de la revendication précédente, dans lequel

- la première huile est une huile marine extraite à partir de coproduits du thon rouge engraissé, cette huile marine extraite étant choisie parmi la liste comprenant l'huile du mésentère, l'huile du foie, l'huile de la vessie natatoire, l'huile de l'estomac, l'huile du coeur, l'huile des reins ;
- la proportion, dans le complément alimentaire, de l'huile d'olive de la variété Chemlali est choisie parmi la liste comprenant

  ◦ 23,7 pour cent en poids du complément alimentaire comprenant l'huile du mésentère ;
  ◦ 26 pour cent en poids du complément alimentaire comprenant l'huile du vessie natatoire ;
  ◦ 16,7 pour cent en poids du complément alimentaire comprenant l'huile du foie ;
  ◦ 47,6 pour cent en poids du complément alimentaire comprenant l'huile de l'estomac ;
  ◦ 41,4 pour cent en poids du complément alimentaire comprenant l'huile du coeur ;
  ◦ 8,3 pour cent en poids du complément alimentaire comprenant l'huile des reins.

7. Le complément alimentaire de l'une quelconque des revendications précédentes, comprenant en outre un agent antioxydant, et/ou un agent anti-goût, et/ou un colorant, et/ou un agent anti-âge.

8. Le complément alimentaire de l'une quelconque des revendications précédentes, étant présenté sous forme d'une capsule, ou sous forme d'une gélule, ou sous forme liquide, ou sous forme de gel liquide.

9. Le complément alimentaire de l'une quelconque des revendications précédentes destiné à être utilisé pour lutter contre l'hypercholestérolémie ou contre les maladies cardiovasculaires, ce complément alimentaire étant appliqué par voie locale, par voie entérale, ou par voie parentérale.

10. Une méthode pour équilibrer la composition en acides gras d'une première quantité d'une première huile, cette première huile ayant un premier score lipidique de prévention supérieur à la teneur en lipides totaux dans cette première huile, cette première huile étant une huile marine, cette méthode comprenant

- une étape de choix d'une deuxième huile ayant un deuxième score lipidique de prévention inférieur à la teneur en lipides totaux dans cette deuxième huile, cette deuxième huile étant une huile végétale et/ou marine ;
- une étape d'ajout d'une deuxième quantité de la deuxième huile à la première quantité de la première huile de sorte que le score lipidique de prévention du mélange de la première quantité et de la deuxième quantité soit sensiblement égale à la teneur, dans ce mélange, en lipides totaux.

11. La méthode de revendication précédente, dans laquelle l'huile marine est choisie parmi la liste comprenant l'huile extraite à partir de coproduits du thon rouge engraissé, l'huile de krill, l'huile de chinchard, l'huile de sardine.

12. La méthode de la revendication précédente, dans laquelle l'huile marine extraite à partir de coproduits du thon rouge engraissé est choisie parmi la liste comprenant l'huile du mésentère, l'huile du foie, l'huile de la vessie natatoire, l'huile de l'estomac, l'huile du coeur, l'huile des reins.

13. La méthode de l'une quelconque des revendications 10 à 12, dans laquelle la deuxième huile comprend

- une huile végétale choisie parmi la liste comprenant l'huile d'olive, l'huile de nigelle, l'huile de mais, l'huile d'amande douce, l'huile de lin, l'huile d'arachide, l'huile de noisette, l'huile de tournesol, l'huile de canola, l'huile de noix; et/ou
- une huile marine choisie parmi la liste comprenant l'huile de calamar, l'huile de foie de morue, l'huile d'anchois.

14. La méthode de la revendication précédente, dans laquelle l'huile d'olive est choisie parmi la liste comprenant l'huile d'olive de la variété Chetoui, l'huile d'olive de la variété Chemlali, l'huile d'olive de la variété Chemchali, l'huile d'olive de la variété Sayali, l'huile d'olive de la variété Meski, l'huile d'olive de la variété Zalmati, l'huile d'olive de la variété Oueslati.

15. La méthode de la revendication précédente, dans laquelle

- la première huile est une huile marine extraite à partir de coproduits du thon rouge engraissé, cette huile marine extraite étant choisie parmi la liste comprenant l'huile du mésentère, l'huile du foie, l'huile de la vessie natatoire, l'huile de l'estomac, l'huile du coeur, l'huile des reins ;
- la deuxième huile est l'huile d'olive de la variété Chemlali, la proportion, dans le mélange de la première quantité de la première huile et de la deuxième quantité de la deuxième huile, est choisie parmi la liste comprenant

∘ 23,7 pour cent en poids du mélange comprenant l'huile du mésentère ;
∘ 26 pour cent en poids du mélange comprenant l'huile du vessie natatoire ;
∘ 16,7 pour cent en poids du mélange comprenant l'huile du foie ;
∘ 47,6 pour cent en poids du mélange comprenant l'huile de l'estomac ;
∘ 41,4 pour cent en poids du mélange comprenant l'huile du coeur ;
∘ 8,3 pour cent en poids du mélange comprenant l'huile des reins.

**Patentansprüche**

1. Nahrungsergänzungsmittel, umfassend:

    - ein erstes Öl mit einem ersten Vorbeugungs-Lipidboniturwert, der höher als der Gesamtlipidgehalt in diesem ersten Öl ist, wobei es sich bei dem ersten Öl um ein Meeresöl handelt;
    - ein zweites Öl mit einem zweiten Vorbeugungs-Lipidboniturwert, der niedriger als der Gesamtlipidgehalt in diesem zweiten Öl ist, wobei es sich bei dem zweiten Öl um ein Pflanzen- und/oder Meeresöl handelt;

    wobei das Nahrungsergänzungsmittel **dadurch gekennzeichnet ist, dass** der Vorbeugungs-Lipidboniturwert der Mischung des ersten Öls und des zweiten Öls im Wesentlichen gleich dem Gesamtlipidgehalt in dieser Mischung ist.

2. Nahrungsergänzungsmittel nach dem vorhergehenden Anspruch, wobei das Meeresöl aus der Aufzählung umfassend aus Beiprodukten von gemästetem Rotem Thun extrahiertes Öl, Krillöl, Stöckeröl, Sardinenöl ausgewählt ist.

3. Nahrungsergänzungsmittel nach dem vorhergehenden Anspruch, wobei das aus Beiprodukten von gemästetem Rotem Thun extrahierte Meeresöl aus der Aufzählung umfassend Mesenteriumöl, Leberöl, Schwimmblasenöl, Magenöl, Herzöl, Nierenöl ausgewählt ist.

4. Nahrungsergänzungsmittel nach einem der vorhergehenden Ansprüche, wobei das zweite Öl Folgendes umfasst:

    - ein Pflanzenöl, ausgewählt aus der Aufzählung umfassend Olivenöl, Schwarzkümmelöl, Maisöl, Süßmandelöl, Leinöl, Erdnussöl, Haselnussöl, Sonnenblumenöl, Canolaöl, Nussöl; und/oder
    - ein Meeresöl, ausgewählt aus der Aufzählung umfassend Kalmaröl, Dorschleberöl, Sardellenöl.

5. Nahrungsergänzungsmittel nach dem vorhergehenden Anspruch, wobei das Olivenöl aus der Aufzählung umfassend Olivenöl der Sorte Chetoui, Olivenöl der Sorte Chemlali, Olivenöl der Sorte Chemchali, Olivenöl der Sorte Sayali, Olivenöl der Sorte Meski, Olivenöl der Sorte Zalmati, Olivenöl der Sorte Oueslati ausgewählt ist.

6. Nahrungsergänzungsmittel nach dem vorhergehenden Anspruch, wobei

    - es sich bei dem ersten Öl um aus Beiprodukten von gemästetem Rotem Thun extrahiertes Meeresöl handelt, wobei das extrahierte Meeresöl aus der Aufzählung umfassend Mesenteriumöl, Leberöl, Schwimmblasenöl, Magenöl, Herzöl, Nierenöl ausgewählt ist;
    - das Verhältnis in diesem Nahrungsergänzungsmittel von Olivenöl der Sorte Chemlali aus der Aufzählung umfassend Folgendes ausgewählt ist:

        ∘ 23,7 Gewichtsprozent des Nahrungsergänzungsmittels umfassend das Mesenteriumöl;
        ∘ 26 Gewichtsprozent des Nahrungsergänzungsmittels umfassend das Schwimmblasenöl;
        ∘ 16,7 Gewichtsprozent des Nahrungsergänzungsmittels umfassend das Leberöl;
        ∘ 47,6 Gewichtsprozent des Nahrungsergänzungsmittels umfassend das Magenöl;
        ∘ 41,4 Gewichtsprozent des Nahrungsergänzungsmittels umfassend das Herzöl;
        ∘ 8,3 Gewichtsprozent des Nahrungsergänzungsmittels umfassend das Nierenöl.

7. Nahrungsergänzungsmittel nach einem der vorhergehenden Ansprüche, umfassend weiterhin ein Antioxidationsmittel und/oder ein Geschmackskaschierungsmittel und/oder einen Farbstoff und/oder ein Mittel gegen das Altern.

8. Nahrungsergänzungsmittel nach einem der vorhergehenden Ansprüche, das in Form einer Kapsel oder in Form einer Gelkapsel oder in flüssiger Form oder in Form eines flüssigen Gels vorliegt.

9. Nahrungsergänzungsmittel nach einem der vorhergehenden Ansprüche zur Verwendung bei der Bekämpfung von Hypercholesterinämie oder gegen Herz-Kreislauf-Krankheiten, wobei das Nahrungsergänzungsmittel auf lokalem Weg, auf enteralem Weg oder auf parenteralem Weg verabreicht wird.

10. Verfahren zum Wiederherstellen des Gleichgewichts der Fettsäurezusammensetzung einer ersten Menge eines ersten Öls, wobei das erste Öl einen Vorbeugungs-Lipidboniturwert aufweist, der höher als der Gesamtlipidgehalt in diesem ersten Öl ist, wobei es sich bei dem ersten Öl um ein Meeresöl handelt, wobei das Verfahren Folgendes umfasst:

   - einen Schritt der Auswahl eines zweiten Öls mit einem zweiten Vorbeugungs-Lipidboniturwert, der niedriger als der Gesamtlipidgehalt in dem zweiten Öl ist, wobei es sich bei dem zweiten Öl um ein Pflanzenöl und/oder ein Meeresöl handelt;
   - einen Schritt der Zugabe einer zweiten Menge des zweiten Öls zu der ersten Menge des ersten Öls so, dass der Vorbeugungs-Lipidboniturwert der Mischung der ersten Menge und der zweiten Menge im Wesentlichen gleich dem Gesamtlipidgehalt in dieser Mischung ist.

11. Verfahren nach dem vorhergehenden Anspruch, wobei das Meeresöl aus der Aufzählung umfassend aus Beiprodukten von gemästetem Rotem Thun extrahiertes Öl, Krillöl, Stöckeröl, Sardinenöl ausgewählt ist.

12. Verfahren nach dem vorhergehenden Anspruch, wobei das aus Beiprodukten von gemästetem Rotem Thun extrahierte Meeresöl aus der Aufzählung umfassend Mesenteriumöl, Leberöl, Schwimmblasenöl, Magenöl, Herzöl, Nierenöl ausgewählt ist.

13. Verfahren nach einem der Ansprüche 10 bis 12, wobei das zweite Öl Folgendes umfasst:

   - ein Pflanzenöl, ausgewählt aus der Aufzählung umfassend Olivenöl, Schwarzkümmelöl, Maisöl, Süßmandelöl, Leinöl, Erdnussöl, Haselnussöl, Sonnenblumenöl, Canolaöl, Nussöl; und/oder
   - ein Meeresöl, ausgewählt aus der Aufzählung umfassend Kalmaröl, Dorschleberöl, Sardellenöl.

14. Verfahren nach dem vorhergehenden Anspruch, wobei das Olivenöl aus der Aufzählung umfassend Olivenöl der Sorte Chetoui, Olivenöl der Sorte Chemlali, Olivenöl der Sorte Chemchali, Olivenöl der Sorte Sayali, Olivenöl der Sorte Meski, Olivenöl der Sorte Zalmati, Olivenöl der Sorte Oueslati ausgewählt ist.

15. Verfahren nach dem vorhergehenden Anspruch, wobei

   - es sich bei dem ersten Öl um aus Beiprodukten von gemästetem Rotem Thun extrahiertes Meeresöl handelt, wobei das extrahierte Meeresöl aus der Aufzählung umfassend Mesenteriumöl, Leberöl, Schwimmblasenöl, Magenöl, Herzöl, Nierenöl ausgewählt ist;
   - es sich bei dem zweiten Öl um Olivenöl der Sorte Chemlali handelt, wobei das Verhältnis in der Mischung der ersten Menge des ersten Öls und der zweiten Menge des zweiten Öls aus der Aufzählung umfassend Folgendes ausgewählt ist:

     ◦ 23,7 Gewichtsprozent des Nahrungsergänzungsmittels umfassend das Mesenteriumöl;
     ◦ 26 Gewichtsprozent des Nahrungsergänzungsmittels umfassend das Schwimmblasenöl;
     ◦ 16,7 Gewichtsprozent des Nahrungsergänzungsmittels umfassend das Leberöl;
     ◦ 47,6 Gewichtsprozent des Nahrungsergänzungsmittels umfassend das Magenöl;
     ◦ 41,4 Gewichtsprozent des Nahrungsergänzungsmittels umfassend das Herzöl;
     ◦ 8,3 Gewichtsprozent des Nahrungsergänzungsmittels umfassend das Nierenöl.

**Claims**

1. A food supplement comprising

   - a first oil having a first lipid prevention score higher than the total lipid content in this first oil, this first oil being a marine oil;
- a second oil having a second lipid prevention score lower than the total lipid content in this second oil, this second oil being a vegetable oil and/or a marine oil;

said food supplement **characterized in that** the sum of the first lipid prevention score and the second lipid prevention score is substantially equal to the total liquid content in the food supplement.

2. The food supplement of the preceding claim, wherein the marine oil is chosen from the group including the extracted oil from the byproducts of fattened bluefin tuna, the krill oil, the mackerel oil, the sardine oil.

3. The food supplement of the preceding claim, wherein the extracted oil from the byproducts of fattened bluefin is chosen from the group including the oil extracted from mesentery, the oil extracted from liver, the oil extracted from swim bladder, the oil extracted from stomach, the oil extracted from heart, the oil extracted from kidneys.

4. The food supplement of any of the preceding claims, wherein the second oil comprises

   - a vegetable oil chosen from the group including nigella oil, corn oil, sweet almond oil, linseed oil, peanut oil, hazelnut oil, sunflower oil, olive oil, canola oil, nut oil; and/or
   - a marine oil chosen from the group including anchovy oil, squid oil, cod liver oil.

5. The food supplement of the preceding claim, wherein the olive oil is chosen from the group including Chetoui olive oil variety, Chemlali olive oil variety, Chemchali olive oil variety, Sayali olive oil variety, Meski olive oil variety, Zalmati olive oil variety, Oueslati olive oil variety.

6. The food supplement of the preceding claim, wherein

   - the first oil is an extracted oil from the byproducts of fattened bluefin, this extracted oil being chosen from the oil extracted from mesentery, the oil extracted from liver, the oil extracted from swim bladder, the oil extracted from stomach, the oil extracted from heart, the oil extracted from kidneys;
   - the proportion, in the food supplement, of the Chemlali olive oil variety is chosen from the group including

     ○ 23,7 percent of the food supplement comprising oil extracted from mesentery ;
     ○ 26 percent of the food supplement comprising oil extracted from swim bladder;
     ○ 16,7 percent of the food supplement comprising oil extracted from liver ;
     ○ 47,6 percent of the food supplement comprising oil extracted from liver ;
     ○ 41,4 percent of the food supplement comprising oil extracted from heart ;
     ○ 8,3 percent of the food supplement comprising oil extracted from kidney.

7. The food supplement of any of the preceding claims, further comprising anti-oxidizing agents, and/or anti-flavors agents, and/or colorants agents, and/or anti-aging agents.

8. The food supplement of any of the preceding claims, wherein it is in the form of capsules, or liquids, or liquid gels.

9. The food supplement of any of the preceding claims intended to be used to fight against hypercholesterolemia, or against cardiovascular diseases, this food supplement being applied locally, enterally or parenterally.

10. Method for balancing, in terms of fatty acids, a first amount of a first oil, this first oil having a first lipid prevention score higher than the total lipid content in this first oil, this first oil being a marine oil, said method comprising

    - a selecting step of a second oil having a second lipid prevention score lower than the total lipid content in this second oil, this second oil being a vegetable oil and/or a marine oil;
    - an adding step of a second amount of the second oil to the first amount of the first oil so that the sum of the first lipid prevention score of the first amount and the second lipid prevention score of the second amount is substantially equal to the total lipid content in the mixture of the first amount of the first oil and the second amount of the second oil.

11. The method of the preceding claim, wherein the marine oil is chosen from the group including the extracted oil from the byproducts of fattened bluefin tuna, the krill oil, the mackerel oil, the sardine oil.

**12.** The method of the preceding claim, wherein the extracted oil from the byproducts of fattened bluefin is chosen from the group including the oil extracted from mesentery, the oil extracted from liver, the oil extracted from swim bladder, the oil extracted from stomach, the oil extracted from heart, the oil extracted from kidneys.

**13.** The method of any of claims 10 to 12, wherein the second oil comprises

- a vegetable oil chosen from the group including nigella oil, corn oil, sweet almond oil, linseed oil, peanut oil, hazelnut oil, sunflower oil, olive oil, canola oil, nut oil; and/or
- a marine oil chosen from the group including anchovy oil, squid oil, cod liver oil.

**14.** The method of the preceding claim, wherein the olive oil is chosen from the group including Chetoui olive oil variety, Chemlali olive oil variety, Chemchali olive oil variety, Sayali olive oil variety, Meski olive oil variety, Zalmati olive oil variety, Oueslati olive oil variety.

**15.** The method of the preceding claim, wherein

- the first oil is an extracted oil from the byproducts of fattened bluefin, this extracted oil being chosen from the oil extracted from mesentery, the oil extracted from liver, the oil extracted from swim bladder, the oil extracted from stomach, the oil extracted from heart, the oil extracted from kidneys;
- the second oil is Chemlali olive oil variety, the proportion, in the mixture of the first amount of the first oil and the second amount of the second oil, is chosen from the group including

  ◦ 23,7 percent of the food supplement comprising oil extracted from mesentery ;
  ◦ 26 percent of the food supplement comprising oil extracted from swim bladder;
  ◦ 16,7 percent of the food supplement comprising oil extracted from liver ;
  ◦ 47,6 percent of the food supplement comprising oil extracted from liver ;
  ◦ 41,4 percent of the food supplement comprising oil extracted from heart ;
  ◦ 8,3 percent of the food supplement comprising oil extracted from kidney.

**EP 2 950 667 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006118463 A1

- WO 2009120091 A1